# EUROPEAN PATENT SPECIFICATION

(11) **EP 4 213 647 B1**
(45) Date of publication and mention of the grant of the patent: **24.07.2024**
(21) Application number: 21787498.1
(22) Date of filing: 17.09.2021
(51) Int. Cl.: A23L 19/00, A23L 33/105, A61K 36/54

(54) **FERMENTED AVOCADO GRANULATE AND FOOD SUPPLEMENTS CONTAINING IT**
FERMENTIERTES AVOCADOGRANULAT UND DIESES ENTHALTENDE NAHRUNGSMITTELZUSATZSTOFFE
GRANULÉ D'AVOCAT FERMENTÉ ET COMPLÉMENTS ALIMENTAIRES LE CONTENANT

(30) Priority: 18.09.2020 IT 202000022102
(43) Date of publication of application: 26.07.2023
(73) Proprietor: PECTEN GROUP S.R.L., 56121 Pisa (IT)
(72) Inventor: MAFFEI, Chiara, 56123 Pisa (IT)
(74) Representative: Cattaneo, Elisabetta
(86) International application number: PCT/IB2021/058497
(87) International publication number: WO 2022/058955

(56) References cited:
- CN-A- 107 853 522
- CN-A- 108 384 675
- US-A1- 2018 369 193
- FILANNINO PASQUALE ET AL: "Lactic acid fermentation enriches the profile of biogenic fatty acid derivatives of avocado fruit (Persea americana Mill.)", FOOD CHEMISTRY, ELSEVIER LTD, NL, vol. 317, 10 February 2020 (2020-02-10), XP086076685, ISSN: 0308-8146, [retrieved on 20200210], DOI: 10.1016/J.FOODCHEM.2020.126384

## Description

### FIELD OF THE INVENTION

The present invention relates to a granulated product based on an avocado fermentate particularly suitable for the production of food supplements and functional foods thanks to its high stability over time.

### STATE OF THE ART

The avocado is the fruit of the *Persea americana Mill.,* a tropical plant native to Mexico in Central America, and is today a food that is increasingly consumed and widespread in the human diet thanks to its high and valuable nutritional content. Because of its unique chemical composition, which is particularly rich in monounsaturated fatty acids (also known as MUFA) and other nutrients such as vitamins, minerals, dietary fibre, phenolic compounds, carotene, thiamine, phytosterols and phytostanols, a diet rich in avocados is associated with hypoglycaemic, antihypertensive, antioxidant, anti-obesity, antimicrobial and liver-protective effects to the benefit of the body. According to a number of recent studies (see for example "The Fertility Diet" by Jorge E. Chavarro, M.D., Walter C. Willett, M.D. and Patrick J. Skerrett, (McGraw-Hill)), avocado is also a food that can play a protective role and stimulate female fertility. FILANNINO PASQUALE ET AL: "Lactic acid fermentation enriches the profile of biogenic fatty acid derivatives of avocado fruit (Persea americana Mill.)",FOOD CHEMISTRY, ELSEVIER LTD, NL, vol. 317, 10 February 2020 and document CN 108 384 675 A disclose fermented avocado compositions having health benefits. Document US 2018/369193 A1 discloses a granular product comprising avocado and maltodextrin.

There is therefore an objective interest in the beneficial properties associated with avocado intake and, thanks to these properties, the avocado is now not only popular as a food but also increasingly being studied as an ingredient in food supplements and functional foods.

Fermentation is also known to lead to a change in the composition of the product, often increasing the concentration of certain nutrients, resulting in fermented products characterized by higher digestibility and greater bioavailability.

Compositions containing an avocado fermentate are therefore also the subjectmatter of interest and study.

CN107853522 describes for example a nutrient solution comprising from 75 to 95 parts of avocado fermentate with protective properties for the uterus.

However, it is also known that fermented products, such as for example avocado fermentates, are typically subject to significant alterations in the composition and chemical-physical properties over time, which reduce the content of their highly bioavailable nutrients, thus compromising the efficacy thereof.

In order to cope with this application and functional limitation, the industry usually resorts to the adoption of special precautions in order to slow down said alterations (e.g. to the addition of stabilising additives), which often lead to an undesirable change in the composition of the products and an increase in production costs, or even limits the use of said fermentates only in short-term final products. In fact, there is therefore a strong technical limitation to the use of fermented products of plant origin, such as avocado fermentates, in products such as food supplements and functional foods, especially in long-term products.

The aim of the present invention is therefore to provide a product based on an avocado fermentate, which can be effectively used in food supplements and functional foods thanks to the ability of not undergoing significant alterations in composition and of maintaining a high content of highly bioavailable nutrients over time.

### SUMMARY OF THE INVENTION

The Applicant has now found that this aim can be achieved by preparing a granulate of an avocado fermentate with a suitable binder.

In particular, in a first aspect thereof the present invention refers to a granulated product comprising an avocado fermentate and at least one binder selected from the group consisting of: a maltodextrin, a cellulose or a derivative thereof, a sugar, a fructooligosaccharide (FOS), acacia, polyvinylpyrrolidone (PVP), and a fibre.

The Applicant has in fact found that by conveniently identifying a final form and by combining the avocado fermentate with a suitable type of binder, it is possible to obtain a product comprising an avocado fermentate capable of ensuring an adequate stability over time, and therefore of being effectively used in food supplements and functional foods by virtue of the absence of significant variations in the composition and chemical-physical properties, which would otherwise reduce the content of highly bioavailable nutrients, thus compromising the efficacy thereof.

In a further aspect thereof, the present invention further refers to a composition comprising the granulated product according to the present invention and at least one suitable food excipient.

In fact, thanks to the increased stability of the granulated product over time according to the present invention, it is in fact possible to use said product by exploiting its increased concentration of nutrients and their greater bioavailability for the preparation of food supplements and functional foods.

In accordance with a further aspect, the present invention therefore also refers to the use of the granulated product or composition according to the present invention as a food supplement or as a functional food.

Furthermore, the present disclosure refers to the use of the granulated product or composition according to the present invention as a substance with a hypoglycaemic effect for the treatment of hyperglycaemia, with an antihypertensive effect for the treatment of hypertension, with an antioxidant effect for the treatment of oxidative stress, with an anti-obesity effect for the treatment of obesity, as an active substance for the antimicrobial treatment and for the liver protection treatment, in a male or female subject, and as an active substance in the treatment for the improvement, restoration, or enhancement of fertility in a male or female subject, as well.

Finally, the present disclosure further refers to the use of the granulated product or composition according to the present invention as an effective substance in improving the general state of health, in reducing symptoms of fatigue and tiredness, for the treatment of premenstrual disorders (abdominal bloating, breast tension, irritability), menstrual pain (dysmenorrhoea), dyspareunia (pain during sexual intercourse) and catamenial headache (menstrual headache), as well as for the treatment of regularisation of the menstrual cycle by reducing its quantity and duration and regularising its rhythm.

The increased concentration of nutrients, and their increased bioavailability in the avocado fermentate, in fact enable the granulated product and composition according to the present invention to show said beneficial effects on the body.

### DETAILED DESCRIPTION OF THE INVENTION

In a first aspect thereof the present invention refers to a granulated product comprising an avocado fermentate and at least one binder selected from the group consisting of: a maltodextrin, a cellulose or a derivative thereof, a sugar, a fructooligosaccharide (FOS), acacia, polyvinylpyrrolidone (PVP), and a fibre.

For the purposes of the present invention, by avocado fermentate it is meant the product obtained by fermenting fresh avocado pulp which may also comprise unfermented fresh avocado pulp (or fractions thereof), usually at a concentration by weight relative to the final fermented product not exceeding 10% by weight.

The Applicant has in fact found that by conveniently identifying a final form and by combining the avocado fermentate with a suitable type of binder, it is possible to obtain a product comprising an avocado fermentate capable of ensuring an adequate stability over time, and therefore of being effectively used in food supplements and functional foods by virtue of the absence of significant variations in the composition and chemical-physical properties, which would otherwise reduce the content of highly bioavailable nutrients, thus compromising the efficacy thereof.

Within the context of the present description and following claims, all the numerical magnitudes indicating quantities, parameters, percentages, and so on are to be considered preceded in every circumstance by the term "about" unless indicated otherwise. Further, all the ranges of numerical magnitudes include all the possible combinations of maximum and minimum numerical values and all the possible intermediate ranges, as well as those indicated below.

Within the scope of the present invention, when reference is made to "avocado", this means the fruit of *Persea americana Mill.*

In this description and in subsequent claims, the term "functional food" is understood to indicate those products, including isolated nutrients, food supplements, herbal products, meal replacements and fresh or transformed foods, that are rich in molecules with beneficial and protective properties for the body, with actions on a nutritional and physiological level.

The present invention can be presented in one or more of its aspects or one or more of the preferred characteristics reported below, which can be combined with one another according to the application requirements.

The granulated product according to the present invention comprises an avocado fermentate and at least one binder selected from the group consisting of: a maltodextrin, a cellulose or a derivative thereof, a sugar, a fructooligosaccharide (FOS), acacia, polyvinylpyrrolidone (PVP), and a fibre.

Preferably the at least one binder is a maltodextrin, more preferably a maize maltodextrin, or the at least one binder is a sugar, preferably isomalt or dextrose, more preferably isomalt.

When the binder is a cellulose derivative, it is preferably methylcellulose, ethylcellulose or carboxymethylcellulose.

The preparation of an avocado fermentate is a technique well known to the person skilled in the art, and within the scope of the present invention, the latter may use any of said fermentation techniques for obtaining an avocado fermentate.

In a preferred embodiment of the present invention, said fermentate is obtained by fermenting fresh avocado pulp, preferably diluted with water, in the presence of at least one fermentation agent selected from the group consisting of: a yeast and a lactobacillus. Preferably, said at least one yeast is of the genus Saccharomyces, even more preferably it is a Saccharomyces boulardii.

Preferably, in the granulated product according to the present invention the weight ratio of said avocado fermentate to said at least one binder ranges from 1:5 to 1:1.

In a preferred embodiment, in the granulated product according to the present invention the weight ratio of said avocado fermentate to said at least one binder ranges from 1:4 to 1:2, more preferably from 1:3.5 to 1 :2.5, optimally about 1:3. In another preferred embodiment, in the granulated product according to the present invention the weight ratio of said avocado fermentate to said at least one binder ranges from 1:2 to 1:1, more preferably from 1:1.5 to 1:1, optimally about 1:1.25.

The Applicant has found that said weight ratios are optimal for obtaining a granulated product that does not undergo significant alterations in composition and preserves a high content of highly bioavailable nutrients over time. Preferably, the granulated product according to the present invention has a particle size such that 100% of the particles have a diameter of less than 2 mm (i.e. pass through a 10 mesh sieve).

Preferably, the granulated product according to the present invention comprises less than 10 % by weight, more preferably from 3 % to 6 % by weight of water.

Among the advantages of the granulated product according to the present invention it is included the fact that it is easy to obtain. In fact, the granulated product according to the present invention is obtainable according to a process that involves a limited number of steps.

Said process includes the following steps:
- preparing an avocado pulp, possibly with added water;
- fermenting an avocado pulp, preferably in the presence of at least one fermentation agent selected from the group consisting of: a yeast and a lactobacillus, so as to obtain an avocado fermentate; and
- granulating in the presence of at least one binder said avocado fermentate.

All of the above steps can be carried out in any manner known to the persons killed in the art, without any particular difficulty, adapting the operating conditions to the specific application requirements of the case.

Intermediate processing steps (e.g. concentration, pasteurisation, filtration, washing, drying) and intermediate storage or conditioning may also be envisaged, in accordance with the usual methods for processing fermented products based on vegetable pulp.

As regards the fermentation of avocado pulp, this is a technique well known to the person skilled in the art, and within the scope of the present invention, the latter may use any of said fermentation techniques for obtaining an avocado fermentate.

Said fermentation of fresh avocado pulp, preferably diluted with water, is a fermentation carried out in the presence of at least one fermentation agent selected from the group consisting of: a yeast, and a lactobacillus. Preferably, said at least one yeast is of the genus Saccharomyces, even more preferably it is Saccharomyces boulardii.

Granulation is also a transformation technique well known to the person skilled in the art, and within the scope of the present invention the skilled in the art can resort to any operating mode, adapting the operating conditions to the specific application needs of the case.

Said granulation step is carried out at a controlled temperature of less than or equal to 40°C in order to prevent phenomena of thermal degradation of the nutrients of the granulated product obtained therefrom.

Among the usable granulation techniques, a fluidised bed technique is preferably used, which allows to obtain a stable granulate which, in addition to being more flowable, is more soluble and therefore increases the bioavailability of the nutrients contained therein.

Optionally and advantageously, at the same time or after the granulation step, the process includes a drying step, in order to regulate the water content of the granulated product obtained.

In a further aspect thereof, the present invention further refers to a composition comprising the granulated product according to the present invention and at least one suitable food excipient.

In fact, thanks to the increased stability of the granulated product over time according to the present invention, it is in fact possible to use said product by exploiting its increased concentration of nutrients and their greater bioavailability for the preparation of food supplements and functional foods.

This stability of the granulate is to be considered exceptional, given that the fermented product subjected to granulation may also contain significant quantities of fresh, unfermented and therefore easily putrescible pulp.

Advantageously, said composition comprises, with respect to its total weight, between 70% and 99% by weight of granulated product.

Preferably, said composition comprises, with respect to the total weight of the composition, from 10% to 30% by weight, preferably from 15% to 25% by weight, of fermented avocado. In a preferred embodiment, said composition comprises from 100 to 1500 mg of fermented avocado.

In a preferred embodiment, the composition according to the present invention comprises at least one plant derivative selected from the group consisting of: a non-granulated fermentate, a pulp, a dehydrated pulp, a juice, preferably a dehydrated pulp.

Preferably, said at least one plant derivative is selected from the group consisting of: a non-granulated avocado fermentate, an avocado pulp, a dehydrated avocado pulp, an avocado juice, more preferably a dehydrated avocado pulp.

Preferably, the composition according to the present invention comprises at least one vitamin, preferably selected from the group consisting of: vitamin D, preferably D3, vitamin B, preferably B6 and/or B12.

Optionally, the composition according to the present invention may contain one or more of the other possible ingredients known in the art for food supplements and functional foods, such as for example flavourings, acidity correctors, e.g. citric acid, anti-caking agents, e.g. silicon dioxide, coenzyme Q-10, emulsifiers, e.g. polyoxyethylene sorbitan monooleate, zinc, zinc gluconate, sweeteners, e.g. sucralose, folic acid, maca root, amino acids such as for example L-arginine, bulking agents, e.g. cellulose and calcium phosphate, stabilisers, e.g. crosslinked sodium carboxymethylcellulose and fatty acids, glazing agents, e.g. polyvinyl alcohol, hydroxy-propyl-methylcellulose, calcium carbonate, and talc, colourants, e.g. chlorophyll and chlorophyllin complexes with copper, and riboflavin.

Said other possible ingredients known in the art may be added at any suitable step in the production of the composition according to the present invention, in manners known to the person skilled in the art for this purpose.

Preferably, the composition according to the present invention is prepared in the form of a capsule, powder, granulate, tablet, dragée, bar paste, syrup, aqueous solution, gel, or cream, depending on the application requirements and the chosen modality of intake.

In a preferred embodiment, the composition according to the present invention is prepared in powder form, preferably in a single-dose sachet format.

Alternatively, it is preferably in tablet form.

Advantageously said powder, granulate or tablet form comprises from 100 to 1500 mg of the fermented avocado of the invention per unit dose.

In a particularly preferred embodiment, the composition according to the present invention is prepared in powder form in a single-dose sachet comprising 400 or 1000 mg of said fermented avocado.

Advantageously, the composition according to the present invention is prepared in powder form in a single-dose sachet comprising the granulated product in a concentration by weight with respect to the total weight of the powder contained in the sachet comprised between 18 and 22%, alternatively comprised between 70 and 75%.

In a further particularly preferred embodiment, the composition according to the present invention is prepared in tablet form comprising 300 mg of said fermented avocado.

Advantageously, when the composition according to the present invention is prepared in tablet form, the granulated product is present in a concentration by weight with respect to the total weight of the powder contained in the sachet comprised between 97 and 99%.

The increased stability over time of the granulated product according to the present invention makes it possible to use said product or the composition containing it by exploiting its increased concentration of nutrients and their increased bioavailability for the preparation of food supplements and functional foods.

In accordance with a further aspect, the present invention therefore also refers to the use of the granulated product or composition according to the present invention as a food supplement or as a functional food.

Disclosed, but not claimed, is the use of the granulated product or composition according to the present invention as a substance with a hypoglycaemic effect for the treatment of hyperglycaemia, with an antihypertensive effect for the treatment of hypertension, with an antioxidant effect for the treatment of oxidative stress, with an anti-obesity effect for the treatment of obesity, as an active substance for the antimicrobial treatment and for the liver protection treatment, in a male or female subject, and as an active substance in the treatment for the improvement, restoration, or enhancement of fertility in a male or female subject, as well.

Disclosed, but not claimed, is the use of the granulated product or composition according to the present invention as an effective substance in improving the general state of health, in reducing symptoms of fatigue and tiredness, for the treatment of premenstrual disorders (abdominal bloating, breast tension, irritability), menstrual pain (dysmenorrhoea), dyspareunia (pain during sexual intercourse) and catamenial headache (menstrual headache), as well as for the treatment of regularisation of the menstrual cycle by reducing its quantity and duration and regularising its rhythm.

The increased concentration of nutrients and their greater bioavailability in the fermented avocado granulate in fact enable the composition according to the present invention to exhibit beneficial effects on female and male fertility.

The invention is now described by means of some examples to be considered for non-limiting illustrating purposes thereof.

### EXPERIMENTAL PART

### EXAMPLE 1 - preparation of the granulated product.

1000 grams of an avocado pulp coming from a Sicilian cultivation were introduced into a stainless steel reaction mixer provided with a stirring system. 400 grams of water and 2 grams of Saccharomyces boulardii yeast were added, keeping the system under stirring and at a controlled temperature in a range comprised between 30 and 40°C until fermentation was complete.

Subsequently, 10 parts by weight of the avocado fermentate thus obtained were fed to a fluidised bed granulation plant where it was granulated at a temperature of 40°C in the presence of 30 parts by weight of maize maltodextrin, resulting in a granulated product with a particle size such that 100% of the particles were less than 2 mm in diameter.

During granulation, the granulated product was dried up to a water content of approximately 4.3 % by weight.

The granulate of the avocado fermentate was then characterised compositionally. The values of the main parameters of interest are shown in Table 1 below.

**Table 1 - Values of some significant parameters of the composition of the granulated product.**

| Parameter | Value |
|---|---|
| Proteins | 0.680±0.078 g/100g |
| Total fat substances | 4.16±0.25 g/100g |
| of which monounsaturated fatty acids (MUFA) | 62.89%±1.59% |
| Dietary fibre (high molecular weight fraction) | 1.60±0.37 g/100g |
| Ashes | 0.43±0.04 g/100g |
| Carbohydrates | 88.79±0.59 g/100g |
| Sugars (Glucose, Sucrose and Maltose) | 1.7394±0.2180 g/100g |
| Dry matter | 95.66±0.38 g/100g |
| Alpha tocopherol | 0.430±0.091 mg/100g |
| Potassium | 1710±170 mg/kg |
| Energy value | 399±3 kcal/100g |

As is evident from the data shown in Table 1, the granulated product contains in its lipid fraction a very high concentration of the beneficial MUFAs, which are particularly valuable in the formulation of supplements and functional foods.

The granulated product thus obtained was then subjected to stability tests over time.

Specifically, the product was subjected to stability tests under the following conditions:
(a) natural: temperature of 25±2°C, relative humidity of 60±5%;
(b) intermediate: temperature of 30±2°C, relative humidity of 65±5%
(c) accelerated: temperature of 40±2°C, relative humidity of 75±5%

The results of the tests under the three different conditions are shown in Tables 2a, 2b and 2c below, respectively.

**Table 2a - results of the stability tests on the granulated product under natural conditions (temperature of 25±2°C, relative humidity of 60±5%).**

| Parameter | Month 0 | Month 1 | Month 3 |
|---|---|---|---|
| Organoleptic | compliant | compliant | compliant |
| Loss on drying LOD (≤6%) | compliant | compliant | compliant |

**Table 2b - results of the stability tests on the granulated product under intermediate conditions (temperature of 30±2°C, relative humidity of 65±5%).**

| Parameter | Month 0 | Month 1 | Month 3 |
|---|---|---|---|
| Organoleptic | compliant | compliant | compliant |
| Loss on drying LOD (≤6%) | compliant | compliant | compliant |

**Table 2c - results of the stability tests on the granulated product under accelerated conditions (temperature of 40±2°C, relative humidity of 75±5%).**

| Parameter | Month 0 | Month 1 | Month 3 |
|---|---|---|---|
| Organoleptic | compliant | compliant | compliant |
| Loss on drying LOD (≤6%) | compliant | compliant | compliant |

As is evident from the data shown in Tables 2a, 2b and 2c, the granulated product showed excellent stability over time under all the experimental conditions tested, thus confirming its possibility of use for the preparation of compositions to be used for the manufacture of supplement products.

### EXAMPLE 2 - preparation of granulated product with different binders.

10 parts by weight of the avocado fermentate obtained according to the fermentation procedure of example 1 were fed to a fluidised bed granulation plant and granulated therein at a temperature of 40 °C in the presence of 30 parts by weight of binder.

Granulation tests were carried out with different binders and their usability for granulating fermented avocado was evaluated based on the observation of obtaining processable granules. In other words, the compliance or non-compliance of the granules obtained was assessed on the basis of their hygroscopicity, flowability and ease of drying; essential parameters for the subsequent processing of the granules.

Table 3 below shows the binders used and the results obtained in the granulation.

**Table 3 - granulation tests of avocado fermentate carried out with different binders.**

| Binder | Granulation result |
|---|---|
| Maize maltodextrin | compliant |
| Isomalt | compliant |
| Polyvinylpyrrolidone (PVP) | compliant |
| Pregelatinised starch | NOT compliant |
| Carboxymethyl cellulose | compliant |
| Alginic acid | NOT compliant |

As is evident from the results obtained, not all binders could be used for the granulation of the avocado fermentate. In the cases of non-compliance, this resulted in jelly-like preparations that were difficult to dry, or very viscous products that were not sufficiently flowable for subsequent manufacture of the preparation.

On the other hand, excellent performance was found in particular when using maize maltodextrin and isomalt.

### EXAMPLE 3 - preparation of a powder composition comprising the granulated product.

The granulated product according to Example 1, having a weight ratio of avocado fermentate to maltodextrin of 1:3, was used for the preparation of a powder containing furthermore inositol, Coenzyme Q10, zinc gluconate, vitamin B6, folic acid, vitamin D3, vitamin B12. The resulting powder was then packaged in single-dose sachets of approximately 5 grams (nominal value) with an average content as shown in Table 4.

**Table 4 - Composition of the powder comprising the granulated product.**

| Component | mg |
|---|---|
| Maize maltodextrin | 3000 |
| Fermented avocado | 1000 |
| inositol | 1000 |
| Coenzyme Q10 | 40 |
| Zinc gluconate (expressed as zinc) | 1.5 |
| Vitamin B6 | 4.6 |
| Folic acid | 0.2 |
| Vitamin D3 | 0.05 |
| Vitamin B12 | 0.0125 |

The single-dose sachet thus prepared was then subjected to stability tests over time. Specifically, the product was subjected to stability tests under the following conditions:
(a) natural: temperature of 25±2°C, relative humidity of 60±5%;
(b) intermediate: temperature of 30±2°C, relative humidity of 65±5%
(c) accelerated: temperature of 40±2°C, relative humidity of 75±5%

The results obtained under the three different experimental conditions are shown in Tables 5a, 5b and 5c below.

**Table 5a - results of the stability tests on the granulated product under natural conditions (temperature of 25±2°C, relative humidity of 60±5%).**

| Parameter | Month 0 | Month 1 | Month 3 | Month 6 | Month 9 | Month 12 |
|---|---|---|---|---|---|---|
| Organoleptic | compliant | compliant | compliant | compliant | compliant | compliant |
| Loss on drying LOD (≤6%) | compliant | compliant | compliant | compliant | compliant | compliant |
| pH (150 ml H₂O) | 3.83 | 3.86 | 3.81 | 3.82 | 3.83 | 3.85 |
| Sachet sealing | 100% | 100% | 100% | 100% | 100% | 100% |

**Table 5b - results of the stability tests on the granulated product under intermediate conditions (temperature of 30±2°C, relative humidity of 65±5%).**

| Parameter | Month 0 | Month 1 | Month 3 | Month 6 |
|---|---|---|---|---|
| Organoleptic | compliant | compliant | compliant | compliant |
| Loss on drying LOD (≤6%) | compliant | compliant | compliant | compliant |
| pH (150 ml H₂O) | 3.83 | 3.85 | 3.82 | 3.83 |
| Sachet sealing | 100% | 100% | 100% | 100% |

**Table 5c - results of the stability tests on the granulated product under accelerated conditions (temperature of 40±2°C, relative humidity of 75±5%).**

| Parameter | Month 0 | Month 1 | Month 3 | Month 6 |
|---|---|---|---|---|
| Organoleptic | compliant | compliant | compliant | compliant |
| Loss on drying LOD (≤6%) | compliant | compliant | compliant | compliant |
| pH (150 ml H₂O) | 3.83 | 3.76 | 3.84 | 3.81 |
| Sachet sealing | 100% | 100% | 100% | 100% |

As is evident from the data in Tables 5a, 5b and 5c, the powder composition comprising the granulated product showed, under all the experimental conditions tested, excellent stability over time, with the values of all the observation parameters perfectly within the required specifications, thus confirming its possibility to formulate the granulated product in a form, such as the powder form in the single-dose sachet format, characterised by a marked ease of absorption, and therefore suitable for marketing.

### EXAMPLE 4 - preparation of a composition in tablet form comprising the granulated product.

The granulated product according to Example 1, having a weight ratio of avocado fermentate to maltodextrin of 1:3, was used for the preparation of tablets of approximately 1.3 grams (nominal value) containing furthermore Coenzyme Q10, zinc gluconate, vitamin B6, folic acid, vitamin D3, Vitamin B12, and having an average content as shown in Table 6.

**Table 6 - Composition of the tablets comprising the granulated product.**

| Component | mg |
|---|---|
| Maize maltodextrin | 900 |
| Fermented avocado | 300 |
| Coenzyme Q10 | 10 |
| Zinc gluconate (expressed as zinc) | 1.5 |
| Vitamin B6 | 4.6 |
| Folic acid | 0.1 |
| Vitamin D3 | 0,025 |
| Vitamin B12 | 0.0125 |

The tablets thus prepared were then subjected to stability tests over time. Specifically, the product was subjected to the following conditions:
(a) natural: temperature of 25±2°C, relative humidity of 60±5%;
(b) intermediate: temperature of 30±2°C, relative humidity of 65±5%
(c) accelerated: temperature of 40±2°C, relative humidity of 75±5%

The results obtained under the three different experimental conditions are shown in Tables 7a, 7b and 7c below.

**Table 7a - results of the stability tests on the granulated product under natural conditions (temperature of 25±2°C, relative humidity of 60±5%).**

| Parameter | Month 0 | Month 1 | Month 3 | Month 6 | Month 9 | Month 12 |
|---|---|---|---|---|---|---|
| Organoleptic | compliant | compliant | compliant | compliant | compliant | compliant |
| Average weight (in the range 1235-1365 mg) | 1308 | 1311 | 1307 | 1312 | 1309 | 1311 |
| Weight uniformity | compliant | compliant | compliant | compliant | compliant | compliant |
| Hardness (in the range 18-20 kg) | 20 kg | 20 kg | 20 kg | 19 kg | 20 kg | 19 kg |
| Disaggregation (less than 30 min) | compliant | compliant | compliant | compliant | compliant | compliant |

**Table 7b - results of the stability tests on the granulated product under intermediate conditions (temperature of 30±2°C, relative humidity of 65±5%).**

| Parameter | Month 0 | Month 1 | Month 3 | Month 6 |
|---|---|---|---|---|
| Organoleptic | compliant | compliant | compliant | compliant |
| Average weight (in the range 1235-1365 mg) | 1308 | 1318 | 1312 | 1321 |
| Weight uniformity | compliant | compliant | compliant | compliant |
| Hardness (in the range 18-20 kg) | 20 kg | 20 kg | 19 kg | 20 kg |
| Disaggregation (less than 30 min) | compliant | compliant | compliant | |

**Table 7c - results of the stability tests on the granulated product under accelerated conditions (temperature of 40±2°C, relative humidity of 75±5%).**

| Parameter | Month 0 | Month 1 | Month 3 | Month 6 |
|---|---|---|---|---|
| Organoleptic | compliant | compliant | compliant | compliant |
| Average weight (in the range 1235-1365 mg) | 1308 | 1315 | 1317 | 1308 |
| Weight uniformity | compliant | compliant | compliant | compliant |
| Hardness (in the range 18-20 kg) | 20 kg | 19 kg | 20 kg | 20 kg |
| Disaggregation (less than 30 min) | compliant | compliant | compliant | compliant |

As is evident from the data shown in Tables 7a, 7b and 7c, the tablet composition comprising the granulated product also showed excellent stability over time, with the values of all the observation parameters perfectly within the required specifications over time, thus confirming its possibility to formulate the avocado fermentate also in this form in order to make available a supplement characterised by the presence of active ingredients endowed with many properties, including therapeutic ones, in a form suitable for marketing and easy to take for subjects in need of this type of supplement.

### EXAMPLE 5 - preparation of a powder composition comprising the granulated product.

For the preparation of the granulated product to be used in this powder composition, the avocado pulp was subjected to the same fermentation and then to the granulation process as described in example 1, wherein the maize maltodextrin was replaced however by isomalt, so as to obtain a weight ratio of avocado fermentate to isomalt of 1:1.25.

This granulate was then used to prepare a powder containing furthermore folic acid, Coenzyme Q10, zinc gluconate, vitamin B6, vitamin B12, vitamin D3, L-arginine and maca root.

The resulting powder was then packaged in single-dose stick-packs of approximately 2.1 grams (nominal value) with an average content as shown in Table 8.

**Table 8 - Composition of the powder comprising the granulated product.**

| Component | mg |
|---|---|
| isomalt | 500 |
| fermented avocado | 400 |
| L-arginine | 200 |
| coenzyme Q10 | 100 |
| zinc gluconate (expressed as zinc) | 10 |
| vitamin B6 | 4.6 |
| folic acid | 0.4 |
| vitamin D3 | 0,025 |
| vitamin B12 | 0.0125 |
| maca root | 100 |
| other excipients (citric acid, amorphous silica, sucralose, flavourings) | q.s. |

This composition proved to be particularly effective for use by male patients.

### EXAMPLE 6 - Clinical trial of the product referred to in example 4 aimed at assessing its effects on the general state of health and on the symptoms of fatigue and tiredness.

In order to test the efficacy of the product and gather useful information on the benefits that the patients exhibited by taking it, a study was carried out on 36 women, aged 33 to 42, who took the product for a period of 1 to 6 months.

Patients were asked to rate how they felt after the period of taking the product in relation to their general state of health and the symptoms of tiredness and fatigue.

In particular, in order to assess the general state of health, they were asked to express how they felt at the end of the treatment cycle and a score from 1 to 4 was given depending on their answer, as follows:
- considerably better → score 4
- better → score 3
- I did not notice any significant differences in the general state of wellbeing → score 2
- worse → score 1

As regards instead to the assessment of tiredness and fatigue symptoms at the end of the treatment, it was asked how they perceived these symptoms by asking them to choose from the following statements, each of which was again in this case matched to a relative score:
- I no longer feel tired and I am also more energetic → score 4
- I don't feel tired any more → score 3
- tiredness has been greatly reduced → score 2
- I did not notice any significant differences → score 1

Table 9 below shows all the scores obtained during the tests.

**Table 9 - Results of the test carried out to check the effects of the product on the patients' general state of health and on their symptoms of tiredness and fatigue.**

| **AGE** | **TREATMENT DURATION (months)** | **GENERAL STATE (1-4)** | **TIREDNESS (1-4)** |
|---|---|---|---|
| 33 | 1 | 4 | 2 |
| 33 | 1 | 4 | 2 |
| 35 | 1 | 3 | 1 |
| 35 | 1 | 3 | 1 |
| 38 | 1 | 2 | 1 |
| 39 | 1 | 4 | 2 |
| 40 | 1 | 3 | 2 |
| 40 | 1 | 4 | 2 |
| 40 | 1 | 4 | 2 |
| 40 | 1 | 4 | 2 |
| 40 | 1 | 3 | 2 |
| 41 | 1 | 3 | 2 |
| 41 | 1 | 3 | 2 |
| 41 | 1 | 3 | 2 |
| 42 | 1 | 4 | 2 |
| 43 | 1 | 2 | 1 |
| 29 | 3 | 2 | 1 |
| 30 | 3 | 3 | 3 |
| 33 | 3 | 3 | 3 |
| 35 | 3 | 2 | 1 |
| 38 | 3 | 4 | 1 |
| 38 | 3 | 4 | 2 |
| 39 | 3 | 4 | 2 |
| 39 | 3 | 3 | 2 |
| 39 | 3 | 3 | 2 |
| 39 | 3 | 3 | 2 |
| 39 | 3 | 3 | 2 |
| 40 | 3 | 4 | 2 |
| 41 | 3 | 4 | 2 |
| 42 | 3 | 4 | 2 |
| 43 | 3 | 2 | 1 |
| 43 | 3 | 3 | 2 |
| 45 | 3 | 4 | 2 |
| 34 | 6 | 3 | 4 |
| 39 | 6 | 3 | 2 |
| 42 | 6 | 4 | 2 |

The processing of the data obtained in these tests, as regards the first observation parameter, highlighted that the product had obtained an average score of 3.3, i.e. an improved general state of health had been recognised by the patient, qualitatively included in the judgement between "improved" and "greatly improved". However, this major improvement was at the same level throughout the treatment period, as the averages calculated for the patients who had taken the product for 1, 3 or 6 months were 3.3, 3.2 and 3.3 respectively, and thus almost constant.

This showed that daily use of the product over a long period of time allows maintaining high benefits for the patient's general state of health.

With regard to the symptoms of fatigue and tiredness, an average score of 1.9 was obtained, when calculated over the entire observation period of the patients.

Much more significant is the calculation of the average score values determined for the three single observation periods, i.e. relative to the patients who had taken the product for 1, 3 or 6 months, which were 1.8, 1.9 and 2.7 respectively; these average values, which were also related to the duration of the treatment period, showed that the efficacy of the product on tiredness and fatigue symptoms was increasing over time. Thus, patients who had continued the treatment for up to 3 and 6 months experienced the greatest benefit from this product in reducing these symptoms, reporting that tiredness had been significantly reduced or that they did not feel tired at all.

### EXAMPLE 7 - Clinical trial of the product referred to in example 4 aimed at assessing its effects on premenstrual disorders and menstrual pain (dysmenorrhoea).

In this case a study was carried out on 30 women who typically complained of premenstrual symptoms and dysmenorrhoea.

The women were aged between 33 and 42 and took the product for a period comprised between 1 and 6 months.

At the end of the treatment, the patients were asked to rate on a scale of 0 to 10 (where 0 corresponded to no improvement and 10 corresponded to a significant improvement in symptoms) how they felt after the period of taking the product, particularly in relation to the typical premenstrual disorders (abdominal bloating, breast tension, irritability) and to menstrual pain (dysmenorrhoea).

Table 10 below shows all the scores obtained during the tests.

**Table 10 - Results of the test carried out to check the effects of the product on premenstrual disorders and dysmenorrhoea.**

| **AGE** | **TREATMENT DURATION (months)** | **PREMENSTRUAL DISORDERS (0-10)** | **MENSTRUAL PAIN (0-10)** |
|---|---|---|---|
| 33 | 1 | 8 | 8 |
| 33 | 1 | 8 | 8 |
| 35 | 1 | 8 | 8 |
| 35 | 1 | 8 | 8 |
| 40 | 1 | 5 | 5 |
| 40 | 1 | 9 | 9 |
| 40 | 1 | 9 | 9 |
| 40 | 1 | 8 | 8 |
| 41 | 1 | 10 | 10 |
| 41 | 1 | 10 | 10 |
| 41 | 1 | 10 | 10 |
| 42 | 1 | 5 | 11 |
| 43 | 1 | 8 | 0 |
| 30 | 3 | 3 | 7 |
| 38 | 3 | 5 | 2 |
| 38 | 3 | 9 | 9 |
| 39 | 3 | 7 | 8 |
| 39 | 3 | 7 | 8 |
| 39 | 3 | 7 | 8 |
| 39 | 3 | 7 | 8 |
| 39 | 3 | 7 | 8 |
| 40 | 3 | 7 | 7 |
| 41 | 3 | 10 | 9 |
| 42 | 3 | 5 | 10 |
| 43 | 3 | 8 | 8 |
| 43 | 3 | 8 | 8 |
| 45 | 3 | 6 | 7 |
| 34 | 6 | 8 | 8 |
| 39 | 6 | 6 | 3 |
| 42 | 6 | 9 | 10 |

The processing of the data obtained in these tests, as regards the first observation parameter, that is premenstrual disorders, highlighted that the product had obtained a very high average score, equal to 7.5 on a scale from 0 to 10. Incidentally, the calculation of the same average score as a function of the period of treatment with the product did not give results that significantly deviated from this value.

Similarly, in the case of the assessment of dysmenorrhoea, the patients expressed themselves even more favourably on average, with an average value of 7.7 on a scale from 0 to 10, again without any particular variability in the result as a function the period of treatment.

The product was therefore effective in reducing symptoms linked to premenstrual disorders as well as in reducing dysmenorrhoea.

### EXAMPLE 8 - Clinical trial of the product referred to in example 4 aimed at assessing its effects on dyspareunia.

In this case a study was carried out on 19 women who typically complained of dyspareunia (i.e. pain during sexual intercourse).

The women were aged between 33 and 42 and took the product for a period comprised between 1 and 6 months.

At the end of the treatment, the patients were asked to rate on a scale of 0 to 10 (where 0 corresponded to no improvement and 10 corresponded to a significant improvement in symptoms) how they felt after the period of taking the product, particularly in relation to pain during sexual intercourse.

Table 11 below shows all the scores obtained during the tests.

**Table 11 - Results of the test carried out to check the effects of the product on dyspareunia.**

| **AGE** | **TREATMENT DURATION (months)** | **DYSPAREUNIA (0-10)** |
|---|---|---|
| 33 | 1 | 8 |
| 33 | 1 | 8 |
| 40 | 1 | 5 |
| 40 | 1 | 9 |
| 40 | 1 | 9 |
| 40 | 1 | 6 |
| 42 | 1 | 5 |
| 43 | 1 | 8 |
| 30 | 3 | 5 |
| 38 | 3 | 5 |
| 39 | 3 | 7 |
| 39 | 3 | 6 |
| 39 | 3 | 6 |
| 39 | 3 | 6 |
| 39 | 3 | 6 |
| 40 | 3 | 6 |
| 41 | 3 | 10 |
| 39 | 6 | 8 |
| 42 | 6 | 8 |

The processing of the data obtained in these tests highlighted that the product again had achieved a very significant average score equal to 6.9; this average score even reached the score of 8 in women who had followed the treatment for 6 months, also highlighting the significant long-term benefits of the product.

The product was therefore effective in reducing the painful symptoms of sexual intercourse (dyspareunia).

### EXAMPLE 9 - Clinical trial of the product referred to in example 4 aimed at assessing its effects on catamenial headache (menstrual headache).

In this case a study was carried out on 22 women who typically complained of catamenial headaches (i.e. menstrual headaches).

The women were aged between 33 and 42 and took the product for a period comprised between 1 and 6 months.

At the end of the treatment, the patients were asked to rate on a scale of 0 to 10 (where 0 corresponded to no improvement and 10 corresponded to a significant improvement in symptoms) how they felt after the period of taking the product, particularly in relation to pain during sexual intercourse.

Table 12 below shows all the scores obtained during the tests.

**Table 12 - Results of the test carried out to check the effects of the product on catamenial headache.**

| **AGE** | **TREATMENT DURATION (months)** | **CATAMENIAL HEADACHE (0-10)** |
|---|---|---|
| 33 | 1 | 8 |
| 33 | 1 | 8 |
| 40 | 1 | 5 |
| 40 | 1 | 9 |
| 40 | 1 | 9 |
| 40 | 1 | 6 |
| 42 | 1 | 5 |
| 43 | 1 | 10 |
| 30 | 3 | 7 |
| 38 | 3 | 8 |
| 39 | 3 | 8 |
| 39 | 3 | 7 |
| 39 | 3 | 7 |
| 39 | 3 | 7 |
| 39 | 3 | 7 |
| 40 | 3 | 7 |
| 41 | 3 | 10 |
| 42 | 3 | 6 |
| 43 | 3 | 8 |
| 43 | 3 | 8 |
| 34 | 6 | 10 |
| 42 | 6 | 8 |

The processing of the data obtained in these tests highlighted that the product had a very high average score, equal to 7.6; this average score even reached the average score of 9 in women who had followed the treatment for 6 months, highlighting also the significant long-term benefits of the product.

The product was therefore also effective in reducing the painful symptoms of catamenial headache.

### EXAMPLE 10 - Clinical trial of the product referred to in example 4 aimed at assessing its effects on the typical observation parameters of menstrual flow (quantity, duration, regularity).

In this case, a study was carried out on 20 women aged between 35 and 42 who took the product for a period comprised between 1 and 6 months.

At the end of the treatment, the patients were asked to rate on a scale of 0 to 10 (where 0 corresponded to no improvement and 10 corresponded to a significant improvement in symptoms) how they felt after the period of taking the product, particularly in relation to the observation parameters.

They were then asked to assess whether the quantity and duration of the cycle had decreased and (for patients who had taken the product for at least 3 months) whether it had become more regular.

Table 13 below shows all the scores obtained during the tests.

**Table 13 - Results of the test carried out to check the effects of the product on the quantity, duration and regularity of the cycle.**

| **AGE** | **TREATMENT DURATION (MONTHS)** | **FLOW QUANTITY (0-10)** | **FLOW DURATION (0-10)** | **CYCLE REGULARITY (0-10)** |
|---|---|---|---|---|
| 35 | 1 | 7 | 7 | / |
| 35 | 1 | 7 | 7 | / |
| 40 | 1 | 4 | 5 | / |
| 40 | 1 | 6 | 6 | / |
| 40 | 1 | 6 | 6 | / |
| 40 | 1 | 7 | 7 | / |
| 42 | 1 | 3 | 3 | / |
| 38 | 3 | 5 | 5 | 10 |
| 38 | 3 | 9 | 9 | 8 |
| 39 | 3 | 8 | 8 | 8 |
| 39 | 3 | 8 | 8 | 8 |
| 39 | 3 | 8 | 8 | 8 |
| 39 | 3 | 8 | 8 | 8 |
| 39 | 3 | 8 | 8 | 8 |
| 40 | 3 | 8 | 8 | 8 |
| 41 | 3 | 9 | 8 | 10 |
| 42 | 3 | 7 | 10 | 10 |
| 43 | 3 | 8 | 10 | 10 |
| 39 | 6 | 8 | 7 | 9 |
| 42 | 6 | 8 | 5 | 10 |

The processing of the data obtained in these tests highlighted that the product had obtained the following average scores for the parameters observed:
- an average score of 7.1 for the effects on flow quantity;
- an average score of 7.2 for the effects on flow duration;
- an average score of 8.8 for the effects on cycle regularity.

In all cases, and therefore for all the observation parameters, the average values calculated as a function of the period of treatment with the product increased with the time of treatment, highlighting values of maximum benefit after 6 months of use, demonstrating that daily use of the product over a long period of time provides fundamental support in regularising the woman's menstrual cycle.

The product was therefore incredibly effective in a plurality of situations, particularly in improving the general state of health, in reducing symptoms of fatigue and tiredness, in reducing premenstrual disorders (abdominal bloating, breast tenderness, irritability), menstrual pain (dysmenorrhea), dyspareunia (pain in sexual intercourse), catamenial headache (menstruation headache), as well as in regularising the menstrual cycle by reducing its quantity and duration and regulating its rhythm.

### EXAMPLE 11 - Clinical trial of the product referred to in example 3 for the assessment of its effects on conception.

About 32 women were enrolled, aged between 35 and 42 classified as infertile (looking for pregnancy for more than two years) with at least one previous failed attempt at assisted reproduction.

The women had all normal menstruation but had AMH (Anti-Müllerian Hormone) values ranging from 2.3 µg/l to 0.89 µg/l.

After 3 months of treatment with the product referred to in example 3 supplemented with 400 µg of folic acid, 63.2% of the women showed a positive outcome to the treatment, with 23.8% of the women conceiving spontaneously while waiting for the PMA treatment to be carried out, and 39.4% having a positive pregnancy test after transfer, with developmental pregnancy in 22% of the cases.

The result of the study highlighted a surprising efficacy of the product according to the invention in treating women with fertility problems.

## Claims

1. A granulated product comprising an avocado fermentate and at least one binder selected from the group consisting of: a maltodextrin, a cellulose or a derivative thereof, a sugar, a fructooligosaccharide (FOS), acacia, polyvinylpyrrolidone (PVP), and a fibre.

2. The granulated product according to claim 1, wherein said at least one binder is a maltodextrin, preferably a corn maltodextrin, or is a sugar, preferably isomalt.

3. The granulated product according to claim 1 or 2, wherein the weight ratio between said avocado fermentate and said at least one binder varies from 1: 5 to 1: 1.

4. The granulated product according to any one of claims 1 to 3, wherein said granulated product has a particle size such that 100% of the particles have a diameter of less than 2 mm.

5. A composition comprising the granulated product according to any one of claims 1 to 4 and at least one suitable food excipient.

6. The composition according to claim 5, wherein said composition comprises, with respect to the total weight of the composition, from 70% to 99% by weight, of granulated product.

7. The composition according to claim 5 or 6, wherein said composition comprises, with respect to the total weight of the composition, from 10% to 30% by weight, preferably from 15% to 25% by weight, of avocado fermentate.

8. The composition according to any one of claims 5 to 7, wherein said composition comprises from 100 to 1500 mg of avocado fermentate.

9. The composition according to any one of claims 5 to 8, comprising at least one vegetable derivative selected from the group consisting of: a non-granulated fermentate, a pulp, a dehydrated pulp, a juice, preferably a dehydrated pulp.

10. The composition according to any one of claims 5 to 9, wherein said at least one vegetable derivative is selected from the group consisting of: a non-granulated avocado fermented, an avocado pulp, a dehydrated avocado pulp, an avocado juice, preferably a dehydrated avocado pulp.

11. The composition according to any one of claims 5 to 10 comprising at least one vitamin, preferably selected from the group consisting of: vitamin D, preferably D3, vitamin B, preferably B6 and / or B12.

12. The composition according to any one of claims 5 to 11, in the form of a capsule, powder, granulate, tablet, dragee, tablet, paste, syrup, aqueous solution, gel, or cream.

13. The composition according to any one of claims 5 to 12, in the form of powder, granulate or tablet comprising a unit dose of from 100 to 1500 mg of avocado fermentate per unit dose.

14. The composition according to claim 13, in the form of a powder in a single-dose sachet format comprising 400 or 1000 mg of avocado fermentate.

15. The composition according to claim 13, in tablet form comprising 300 mg of avocado fermentate.

16. The granulated product according to any of claims 1 to 4, or the composition according to any of claims 5 to 15, for use as a food supplement or as a functional food.

## Patentansprüche

1. Granuliertes Produkt, das ein Avocadofermentat und mindestens ein Bindemittel umfasst, ausgewählt aus der Gruppe, bestehend aus: einem Maltodextrin, einer Cellulose oder einem Derivat davon, einem Zucker, einem Fructooligosaccharid (FOS), Akazie, Polyvinylpyrrolidon (PVP) und einer Faser.

2. Granuliertes Produkt nach Anspruch 1, wobei das mindestens eine Bindemittel ein Maltodextrin, vorzugsweise ein Maismaltodextrin, oder ein Zucker, vorzugsweise Isomalt, ist.

3. Granuliertes Produkt nach Anspruch 1 oder 2, wobei das Gewichtsverhältnis zwischen dem Avocadofermentat und dem mindestens einen Bindemittel zwischen 1:5 und 1:1 liegt.

4. Granuliertes Produkt nach einem der Ansprüche 1 bis 3, wobei das granulierte Produkt eine derartige Partikelgröße aufweist, dass 100% der Partikel einen Durchmesser von weniger als 2 mm aufweisen.

5. Zusammensetzung, die das granulierte Produkt nach einem der Ansprüche 1 bis 4 und mindestens einen geeigneten Nahrungsmittelhilfsstoff umfasst.

6. Zusammensetzung nach Anspruch 5, wobei die Zusammensetzung, bezogen auf das Gesamtgewicht der Zusammensetzung, 70 Gew.-% bis 99 Gew.-% des granulierten Produkts umfasst.

7. Zusammensetzung nach Anspruch 5 oder 6, wobei die Zusammensetzung, bezogen auf das Gesamtgewicht der Zusammensetzung, 10 Gew.-% bis 30 Gew.-%, vorzugsweise 15 Gew.-% bis 25 Gew.-%, Avocadofermentat umfasst.

8. Zusammensetzung nach einem der Ansprüche 5 bis 7, wobei die Zusammensetzung 100 bis 1500 mg Avocadofermentat umfasst.

9. Zusammensetzung nach einem der Ansprüche 5 bis 8, die mindestens ein pflanzliches Derivat umfasst, ausgewählt aus der Gruppe, bestehend aus: einem nicht granulierten Fermentat, einer Pulpe, einer dehydrierten Pulpe, einem Saft, vorzugsweise einer dehydrierten Pulpe.

10. Zusammensetzung nach einem der Ansprüche 5 bis 9, wobei das mindestens eine pflanzliche Derivat ausgewählt ist aus der Gruppe, bestehend aus: einem nicht granulierten Avocadofermentat, einer Avocadopulpe, einer dehydrierten Avocadopulpe, einem Avocadosaft, vorzugsweise einer dehydrierten Avocadopulpe.

11. Zusammensetzung nach einem der Ansprüche 5 bis 10, die mindestens ein Vitamin umfasst, vorzugsweise ausgewählt aus der Gruppe, bestehend aus: Vitamin D, vorzugsweise Vitamin D3, Vitamin B, vorzugsweise Vitamin B6 und/oder Vitamin B12.

12. Zusammensetzung nach einem der Ansprüche 5 bis 11, die in Form einer Kapsel, eines Pulvers, eines Granulats, einer Tablette, eines Dragees, einer Pastille, einer, Paste, eines Sirups, einer wässrigen Lösung, eines Gels oder einer Creme vorliegt.

13. Zusammensetzung nach einem der Ansprüche 5 bis 12, die in der Form eines Pulvers, Granulats oder einer Tablette vorliegt, das/die eine Einheitsdosis von 100 bis 1500 mg Avocadofermentat pro Einheitsdosis umfasst.

14. Zusammensetzung nach Anspruch 13, die in der Form eines Pulvers in einem Einzeldosis-Beutelformat vorliegt, das 400 oder 1000 mg Avocadofermentat umfasst.

15. Zusammensetzung nach Anspruch 13, die in Tablettenform vorliegt, die 300 mg Avocadofermentat umfasst.

16. Granuliertes Produkt nach einem der Ansprüche 1 bis 4 oder die Zusammensetzung nach einem der Ansprüche 5 bis 15 zur Verwendung als Nahrungsergänzungsmittel oder als funktionelles Lebensmittel.

## Revendications

1. Produit granulé comprenant un produit de fermentation d'avocat et au moins un liant choisi dans le groupe constitué par : une maltodextrine, une cellulose ou un dérivé de celle-ci, un sucre, un fructooligosaccharide (FOS), de l'acacia, de la polyvinylpyrrolidone (PVP) et une fibre.

2. Produit granulé selon la revendication 1, dans lequel ledit au moins un liant est une maltodextrine, de préférence une maltodextrine de maïs, ou est un sucre, de préférence l'isomalt.

3. Produit granulé selon la revendication 1 ou 2, dans lequel le rapport pondéral entre ledit produit de fermentation d'avocat et ledit au moins un liant varie de 1: 5 à 1: 1.

4. Produit granulé selon l'une quelconque des revendications 1 à 3, dans lequel ledit produit granulé présente une taille de particules telle que 100 % des particules ont un diamètre inférieur à 2 mm.

5. Composition comprenant le produit granulé selon l'une quelconque des revendications 1 à 4 et au moins un excipient alimentaire approprié.

6. Composition selon la revendication 5, dans laquelle ladite composition comprend, par rapport au poids total de la composition, de 70 % à 99 % en poids de produit granulé.

7. Composition selon la revendication 5 ou 6, dans laquelle ladite composition comprend, par rapport au poids total de la composition, de 10 % à 30 % en poids, de préférence de 15 % à 25 % en poids, de produit de fermentation d'avocat.

8. Composition selon l'une quelconque des revendications 5 à 7, dans laquelle ladite composition comprend de 100 à 1500 mg de produit de fermentation d'avocat.

9. Composition selon l'une quelconque des revendications 5 à 8, comprenant au moins un dérivé végétal choisi dans le groupe constitué par : un produit de fermentation non granulé, une pulpe, une pulpe déshydratée, un jus, de préférence une pulpe déshydratée.

10. Composition selon l'une quelconque des revendications 5 à 9, dans laquelle ledit au moins un dérivé végétal est choisi dans le groupe constitué par : un avocat non granulé fermenté, une pulpe d'avocat, une pulpe d'avocat déshydratée, un jus d'avocat, de préférence une pulpe d'avocat déshydratée.

11. Composition selon l'une quelconque des revendications 5 à 10, comprenant au moins une vitamine, de préférence choisie dans le groupe constitué par : la vitamine D, de préférence D3, la vitamine B, de préférence B6 et / ou B12.

12. Composition selon l'une quelconque des revendications 5 à 11, sous la forme d'une capsule, d'une poudre, d'un granulé, d'un comprimé, d'une dragée, d'un comprimé, d'une pâte, d'un sirop, d'une solution aqueuse, d'un gel ou d'une crème.

13. Composition selon l'une quelconque des revendications 5 à 12, sous forme de poudre, de granulé ou de comprimé comprenant une dose unitaire allant de 100 à 1500 mg de produit de fermentation d'avocat par dose unitaire.

14. Composition selon la revendication 13, sous forme d'une poudre en format de sachet unidose comprenant 400 ou 1000 mg de produit de fermentation d'avocat.

15. Composition selon la revendication 13, sous forme de comprimé comprenant 300 mg de produit de fermentation d'avocat.

16. Produit granulé selon l'une quelconque des revendications 1 à 4, ou la composition selon l'une quelconque des revendications 5 à 15, pour une utilisation en tant que complément alimentaire ou en tant qu'aliment fonctionnel.
